# EUROPEAN PATENT SPECIFICATION

(11) **EP 4 289 960 B1**
(45) Date of publication and mention of the grant of the patent: **15.07.2026**
(21) Application number: 22758920.7
(22) Date of filing: 24.02.2022
(51) Int. Cl.: C12N 15/70, C12N 9/52, C12N 15/63, C07K 1/34, C07K 1/14, C07K 19/00, C07K 14/33

(54) **PREPARATION METHOD FOR MODIFIED TOXIN POLYPEPTIDE**
VERFAHREN ZUR HERSTELLUNG EINES MODIFIZIERTEN TOXINPOLYPEPTIDS
PROCÉDÉ DE PRÉPARATION D'UN POLYPEPTIDE DE TOXINE MODIFIÉ

(30) Priority: 26.02.2021 CN 202110217756
(43) Date of publication of application: 13.12.2023
(73) Proprietor: Chongqing Claruvis Pharmaceutical Co., Ltd., Chongqing 400713 (CN)
(72) Inventor: ZHANG, Yan, Chongqing 400722 (CN)
(74) Representative: HGF
(86) International application number: PCT/CN2022/077637
(87) International publication number: WO 2022/179556

(56) References cited:
- WO-A2-2019/118974
- CN-A- 107 438 670
- CN-A- 107 548 402
- CN-A- 110 072 880
- US-A1- 2010 041 098
- US-A1- 2011 092 682
- US-A1- 2012 245 324
- US-A1- 2018 073 089
- US-A1- 2018 080 016

## Description

### PRIORITY

The present application requires priority to Application No. 202110217756.1, filed Feb. 26, 2021.

### TECHNICAL FIELD

The present invention relates to the field of biotechnology and bioengineering, and particularly, to a method for preparing a modified toxin polypeptide.

### BACKGROUND

Polypeptides are a group of compounds formed by the connection of a plurality of amino acids through peptide bonds, generally consisting of 10-100 amino acid molecules, with the same connection manner as proteins and a relative molecular mass of less than 10000. Polypeptides are very common in organisms. Tens of thousands of polypeptides have been found in organisms so far, which are widely involved in regulating the functional activities of various systems, organs, tissues, and cells in the organisms, playing an important role in life activities.

Polypeptide drugs refer to polypeptides with specific therapeutic effects, acquired from chemical synthesis and gene recombination or extracted from animals and plants, and consist of a specific application of polypeptides in the field of pharmaceuticals. Polypeptides have broad and important bioactivities, and can be widely applied to the endocrine system, immune system, digestive system, cardiovascular system, blood vessel system, musculoskeletal system, and the like. As such, the development of polypeptide as drugs has a short history but a fast pace, and has become a hot spot in the market at present. Polypeptides are mainly used for treating serious diseases related to cancers and metabolic disorders, and drugs related to such diseases have fairly important markets all over the world.

Toxin polypeptides occupy a certain share of the polypeptide drugs, including native toxin polypeptides or modified toxin polypeptides produced by genetic recombination. Such toxin polypeptides are well known for their effects in spasm treatment, cosmetology, and antitumor therapies as native molecules or fusion proteins.

Clostridial neurotoxins (CNTs) are known to include seven different serotypes of botulinum neurotoxins (BoNTs) and tetanus toxin (tetX, or tetanus neurotoxin, TeNT), all of which comprise two polypeptide molecules linked by disulfide bonds: a light chain (L) of about 50 KDa and a heavy chain (H) of about 100 KDa. The light chain comprises a protease active region and the heavy chain comprises a translocation domain (N terminus) and a receptor-binding domain (C terminus). All serotypes of botulinum neurotoxins (BoNTs) and tetanus toxins (tetX) produced by *Clostridium tetani* are Zn²⁺ proteases. They prevent synaptic exocytosis, inhibit neurotransmitter release, and interrupt nerve signaling by cleaving a protein involved in the formation of SNARE complex that controls cell membrane fusion. Botulinum neurotoxin type A (BoNT/A) has been approved for the treatment of strabismus, blepharospasm, and other diseases in 1989 in the United States. In some applications, the botulinum toxin is injected directly into the muscles to be treated along with an additional bacterial protein in the form of a complex, and the toxin is released from the protein complex at the physiological pH (Eisele et al., 2011, Toxicon 57 (4): 555-65.) to exert the desired pharmacological effect. The FDA has also approved botulinum neurotoxin type B for the treatment of cervical dystonia.

Diphtheria toxin and pseudomonas exotoxin are commonly used to construct toxin fusion protein-based targeted drugs. The native diphtheria toxin consists of 535 amino acids, with two subunits linked by two disulfide bonds connected to a loop of 14 amino acids. The two subunits of diphtheria toxin comprise three protein domains: a protease domain, a receptor-binding domain, and a translocation domain. Once the protease domain enters the cytoplasm, it prolongs the ADP-ribosylation effect of catalytic factor-2, resulting in the inhibition of protein synthesis and cell death. Therefore, it has been reported that the native receptor-binding domain of diphtheria toxin can be replaced with a receptor-binding domain targeting a tumor cell by genetic engineering, and the fusion protein exerts the toxicity of diphtheria toxin through targeting a specific cell ("Targeting Killing Effect of IL-13 Diphtheria Toxin Fusion Protein and DT389-hIL13-13E13K", Du Juan et al., Journal of Medical Research, 2008 (037) 010, 31-36).

Pseudomonas exotoxin is structurally similar to diphtheria toxin and also has three domains: a protease domain, a receptor-binding domain, and a translocation domain. At present, a series of immune fusion toxins using a pseudomonas toxin as the killing group have been constructed, and interleukins, growth factors, single-chain antibodies, and the like are used as specific ligands (Purification and Renaturation of Recombinant Human Interleukin 2-Pseudomonas Exotoxin (IL2-PE66^{4Glu}) Fusion Protein, Hu Zhiming, et al., Journal of First Military Medical University, 1000-2588 (2002) 03-0206-02).

Cholera toxin (CTX) is a toxin polypeptide produced by *Vibrio cholerae,* which causes serious diarrhea and dehydration in humans. CTX is an oligo-protein with a molecular weight of about 84 KDa, consisting of one A subunit and 5 B subunits surrounding the A subunit. The B subunits are responsible for recognizing and binding the holotoxin to the GM1 ganglioside receptor on the surface of a mammalian cell, and promote the entrance of the A subunit into the cell; the A subunit bears ADP-ribosyl-transferase activity, which down-regulates Gs protein expression and activates AC enzyme, thereby promoting an increase in cAMP level. The A subunit also can ADP-ribosylate the transporter of the outer segment membrane disc of rod cells to inactivate GTPase. Due to the ubiquity of the GM1 ganglioside receptor on eukaryotic cell membranes, CTX is used in a variety of model systems to activate adenylate cyclase (AC). CTX is also a mucosal vaccine adjuvant that induces immune responses of type 2 helper T cells by inhibiting IL-12 production. The fusion protein prepared by utilizing the protease function of the A subunit of the cholera toxin and the targeting recognition and binding functions of the B subunits can be applied to the antitumor treatment field (CN201910673683.X).

Although the role of toxin polypeptides in medical and cosmetic aspects and the role of the fusion protein thereof in antitumor therapies have been recognized, the toxicity of toxin polypeptides makes it necessary to consider environmental reservation and operator protection during the preparation process, and thus the preparation of the toxin polypeptide is usually carried out in a BL-3 environment or in a large-scale isolator, which undoubtedly increases the production cost, and is unfavorable for industrial mass production of such toxins.

US 2018/073089A1 relates to recombinant clostridial neurotoxins which have been modified to improve their activation. US 2018/080016A1 discloses a BoNT poly-peptide with a modified receptor bingding domain having one or more amino acid mutations that modify the binding of the BoNT to the receptor. US 2012/245324A1 discloses a rapid, animal protein free, chromatographic processes and systems for obtaining high potency, high yield botulinum neurotoxin for research, therapeutic and cosmetic use. US 2011/092682A1 discloses a method and system for chromatographically purifying a botulinum neurotoxin. However, none of the prior arts have disclosed the introduction of exogenous proteases into toxin peptide precursors to address production safety issues.

### SUMMARY

In order to overcome the defects in the prior art, the present invention provides a method for preparing a modified toxin polypeptide, specifically as follows:
In a first aspect of the present invention, a method for preparing a toxin polypeptide is provided, comprising: step 1), expressing a toxin polypeptide precursor; step 2), enriching the toxin polypeptide precursor; and step 3), activating the toxin polypeptide precursor to obtain the toxin polypeptide;
wherein the toxin polypeptide precursor comprises:
   (I) the first polypeptide fragment, comprising:
      (a) the tag protein;
      (b) the structural region comprising the first protease cleavage site;
      (c) the short linker peptide;
   (II) the second polypeptide fragment, comprising:
      (d) the first functional amino acid structural region, comprising the metal ion-dependent protease activity domain;
      (e) the structural region comprising the second protease cleavage site;
      (f) the second functional amino acid structural region, comprising the receptor-binding domain that can bind to the surface receptor of the target cell and/or the translocation domain that can mediate the transfer of the polypeptide across the vesicle membrane;
wherein the protease that specifically recognizes the first protease cleavage site and the protease that specifically recognizes the second protease cleavage site are both the proteases of rhinoviruses;
wherein the first protease cleavage site and the second protease cleavage site are both LEVLFQGP;
wherein the second enzyme cleavage site is embedded into, or partially replaces or completely replaces a natural loop region between a first functional peptide fragment and a second functional peptide fragment.

Preferably, the step 1) comprises: (1) constructing a nucleic acid molecule encoding the toxin polypeptide precursor; (2) constructing a vector comprising the nucleic acid molecule; (3) transferring the nucleic acid vector into a suitable host cell; and (4) culturing the host cell, and allowing or inducing the host cell to express the toxin polypeptide precursor encoded by the nucleic acid vector.

More preferably, the step 1) further comprises: (5) fermenting a cell capable of expressing the toxin polypeptide precursor.

Even more preferably, the step 1) further comprises: (6) lysing the cell expressing the toxin polypeptide to obtain a cell lysate comprising the toxin polypeptide precursor.

Preferably, the step 2) comprises enriching the toxin polypeptide precursor by conducting a multiplex filtration procedure.

More preferably, the multiplex filtration comprises a crude liquid filtration and a feed liquid circulation filtration.

Even more preferably, the crude liquid filtration step separates the lysate into a feed liquid and a waste residue.

Even more preferably, a material of the crude liquid filtration has a pore size of 0.1-0.65 µm.

Even more preferably, substances that cannot pass through the crude liquid filtration are discharged as the waste residue.

Even more preferably, the waste residue is separated from the material of the crude liquid filtration under the effect of a buffer.

Preferably, the substance entering the crude liquid filtration is switched from the lysate to the buffer when the filtration rate of the crude liquid is lower than 50% of the initial rate.

Even more preferably, the feed liquid circulation filtration step filters the feed liquid multiple times. In the process of multiple filtering, the feed liquid that meets the requirement of the finished liquid collection is collected as the finished liquid, and the feed liquid that meets the requirement of the waste liquid is discharged as the waste liquid.

Even more preferably, the requirement of finished liquid collection is the turbidity of the feed liquid, and when OD₆₀₀ < 0.1, the feed liquid is collected as the finished liquid.

Even more preferably, the requirement of waste feed liquid discharge is the turbidity of the feed liquid, and when OD₆₀₀ > 0.1, the feed liquid is discharged as the waste feed liquid.

Even more preferably, the requirement of waste feed liquid discharge is that the feed liquid does not meet the requirement of the finished liquid collection after 2 or more cycles of circulation.

Even more preferably, the material of the crude liquid filtration and the material of the feed liquid circulation filtration are selected from a hydrophilic filtration material or a hydrophobic filtration material.

Even more preferably, the hydrophilic filtration material is selected from a cellulose ester, polyethersulfone, polyethylene or the like, or derivatives thereof.

Even more preferably, the hydrophobic filtration material is selected from polyvinylidene fluoride, polypropylene, polytetrafluoroethylene or the like, or derivatives thereof.

More preferably, a lysate separation device is enclosed during operation.

Even more preferably, the lysate separation device does not require an additional isolation measure, for example, an isolator.

Preferably, the step 3) comprises digesting the toxin polypeptide precursor by using a protease to obtain the modified toxin polypeptide.

More preferably, the toxin polypeptide is present in the form of the toxin polypeptide precursor with low toxicity in a cell or a lysate, and the toxin polypeptide with high toxicity is obtained after a protease activation step.

Even more preferably, the low toxicity is relative to the high toxicity after the activation. In a specific embodiment, the activity of the toxin polypeptide after the activation is at least 5000 folds higher than that of the toxin polypeptide precursor, and still further, the activity of the toxin polypeptide after the activation is 6000, 7000, 8000, 9000, 10000, 12000, 15000 or more folds higher than that of the toxin polypeptide precursor.

Preferably, the method further comprises step 4), purifying the toxin polypeptide.

More preferably, the step 4) comprises at least one chromatography procedure. Even more preferably, the step 4) may comprise 2, 3, or more chromatography procedures.

More preferably, the chromatography procedures can be identical or different.

More preferably, the chromatography procedure is affinity chromatography, gel filtration chromatography, and/or ion chromatography.

More preferably, the activation procedure of the step 3) and the purification procedure of step 4) are conducted in an isolator.

More preferably, the metal ion-dependent protease activity domain is a Zn²⁺-dependent protease activity domain.

More preferably, the first functional amino acid structural region and/or the second functional amino acid structural region are encoded by a natural sequence and/or an artificially synthesized sequence.

Even more preferably, the first functional amino acid structural region of the toxin polypeptide precursor comprises a Zn²+ protease binding domain of the light chain of clostridial neurotoxin.

Even more preferably, a target cell of the receptor-binding domain capable of binding to an antigen on the surface of a human cell in the second functional amino acid structural region refers to a target cell with an SNARE complex. For example, a nerve cell, a pancreatic cell, or other cells with an SNARE complex.

Even more preferably, the target cell of the second functional amino acid structural region refers to a human nerve cell or a pancreatic cell, and the receptor-binding domain is a receptor-binding domain capable of specifically binding to the human nerve cell or the pancreatic cell.

Even more preferably, the receptor-binding domain of the second functional amino acid structural region is a cell surface antigen-binding domain of the heavy chain of clostridial neurotoxin, and the translocation domain of the second functional amino acid structural region that mediates the transfer of the polypeptide across the vesicle membrane is a domain that mediates the transfer of clostridial neurotoxin across the vesicle membrane.

Even more preferably, the clostridial neurotoxin is a botulinum neurotoxin or tetanus toxin.

Even more preferably, the botulinum neurotoxin is selected from any one of serotypes BoNT/A to BoNT/H and derivatives thereof known in the art.

Even more preferably, the clostridial neurotoxin is selected from any one of tetanus toxin or a derivative thereof. More preferably, the first functional amino acid structural region comprises part or all of the light chains of BoNT/A, BoNT/B, BoNT/C, BoNT/D, BoNT/E, BoNT/F, BoNT/G, BoNT/H or tetanus toxin.

Even more preferably, the second functional amino acid structural region comprises part or all of the heavy chains of BoNT/A, BoNT/B, BoNT/C, BoNT/D, BoNT/E, BoNT/F, BoNT/G, BoNT/H or tetanus toxin.

In one specific embodiment, the first functional amino acid structural region is the light chain of BoNT/A, and the second functional amino acid structural region is the heavy chain of BoNT/A.

Even more preferably, the first functional amino acid structural region and the second functional amino acid structural region may be from different serotypes. The first functional amino acid structural region and the second functional amino acid structural region may be any combination of the above serotypes. For example, the first functional amino acid structural region is derived from the light chain of BoNT/A and the second functional amino acid structural region is derived from the heavy chain of BoNT/B, or the first functional amino acid structural region is derived from the light chain of BoNT/A and the second functional amino acid structural region is derived from the heavy chain of BoNT/C, and the like.

Even more preferably, the second polypeptide fragment has an amino acid sequence comprising a fragment set forth in SEQ ID NO: 7.

The term "tag protein" refers to a class of protein molecules that can bind to a specific ligand.

The "tag protein" can increase the solubility of the toxin polypeptide precursor in a host.

The "tag protein" allows the presence of the toxin polypeptide precursor in a host cell in a soluble manner.

More preferably, the tag protein is selected from a tag protein known to those skilled in the art or a tag protein designed by a computer program, and is capable of specifically binding to a known substrate.

Even more preferably, the tag protein is selected from, but not limited to, the following proteins: a glutathione S-transferase (GSTs), C-myc, chitin-binding domain, maltose-binding protein (MBP), SUMO heterologous affinity moiety, monoclonal antibody or protein A, streptavidin-binding protein (SBP), cellulose-binding domain, calmodulin-binding peptide, S tag, Strep tag II, FLA, protein A, protein G, histidine affinity tag (HAT), or polyhistidine.

In a specific embodiment, the tag protein is located at an N terminus of the toxin polypeptide precursor.

In a specific embodiment, the tag protein is a glutathione S-transferase (GSTs), and more preferably, the amino acid sequence of the glutathione S-transferase is set forth in SEQ ID NO: 2.

More preferably, neither the first protease cleavage site nor the second protease cleavage site can be cleaved by a human protease or a protease produced by the host cell expressing the toxin polypeptide precursor.

The first protease cleavage site and the second protease cleavage site are not recognized and cleaved by the host cell when being expressed or by an endogenous protease of an organism when being used.

More preferably, the activation of the step 3) comprises: digesting the second protease cleavage site in the toxin polypeptide precursor by using a second protease.

Even more preferably, the activation further comprises: digesting the first protease cleavage site in the toxin polypeptide precursor by using a first protease.

The embedding refers to an embedding between two certain amino acids in the loop region; the partial replacement refers to that the second enzyme cleavage site replaces part of the amino acid sequence of the loop region; the complete replacement refers to that the amino acid sequence of the natural loop region is completely replaced by the second enzyme cleavage site.

More preferably, the short linker peptide has no more than 5 amino acids.

Even more preferably, the short linker peptide enables the first protease cleavage site to be more easily recognized or bound to by a protease thereof.

Even more preferably, the short linker peptide does not affect the function of the toxin polypeptide precursor.

Even more preferably, the short linker peptide retained at the N terminus of the second polypeptide fragment does not affect the function of the second polypeptide fragment after the cleavage of the first protease cleavage site.

Even more preferably, a GS part of the short linker peptide has no more than 5 amino acid residues; more preferably, the short linker peptide is selected from a glycine-serine (GS for short) short peptide, GGS, GGGS, GGGGS, GSGS, GGSGS, GSGGS, GGSGS, GGGSS, and other short linker peptides.

In a specific embodiment, the amino acid sequence of the structural region comprising the first protease cleavage site and the short linker peptide is LEVLFQGPLGS.

In a specific embodiment, the toxin polypeptide precursor sequentially comprises, from the N terminus: the glutathione S-transferase, LEVLFQGPLGS, the light chain of BoNT/A, LEVLFQGP, and the heavy chain of BoNT/A.

More preferably, the toxin polypeptide precursor has an amino acid sequence set forth in SEQ ID NO: 11.

Preferably, the nucleic acid molecule in step (1), constructing a nucleic acid molecule encoding the toxin polypeptide precursor, wherein the nucleic acid molecule is a nucleic acid molecule encoding the toxin polypeptide precursor described above. More preferably, the nucleic acid molecule consists of a nucleotide sequence encoding various parts of the toxin polypeptide precursor.

In a specific embodiment, the sequence encoding the tag protein comprises a sequence set forth in SEQ ID NO: 1.

In a specific embodiment, the nucleotide sequence encoding the structural region comprising the first protease cleavage site comprises a sequence set forth in SEQ ID NO: 3.

In a specific embodiment, the nucleotide sequence encoding the structural region comprising the second protease cleavage site comprises a sequence set forth in SEQ ID NO: 8.

In a specific embodiment, the amino acid sequence of the second polypeptide fragment comprises a sequence set forth in SEQ ID NO: 7.

In a specific embodiment, the nucleotide sequence encoding the second polypeptide fragment comprises a sequence set forth in SEQ ID NO: 6.

In a specific embodiment, the nucleotide sequence encoding the toxin polypeptide precursor is set forth in SEQ ID NO: 10.

Preferably, in step (2), constructing a vector comprising the nucleic acid molecule, the vector comprises the nucleic acid molecule described above or an open reading frame encoding the toxin polypeptide precursor described above.

More preferably, the vector is a plasmid, a phage, a viral vector, or the like.

Preferably, in step (3), transforming the nucleic acid vector into a suitable host cell, the cell is a eukaryotic cell or a prokaryotic cell.

More preferably, the cell is selected from *Escherichia coli,* a yeast, cyanobacterium or a mammalian cell, an insect cell, a plant cell, or an amphibian cell.

Even more preferably, the cell is *Escherichia coli.*

Compared with a conventional method for preparing a toxin polypeptide, in the method of the present invention, the toxin polypeptide is expressed as a toxin polypeptide precursor with low toxicity firstly, and activated to become an active toxin polypeptide after enrichment in a large volume, such that the harm of procedures in a large volume to the environment and workers is greatly reduced, and the cost of isolation equipment is reduced. The enrichment procedure of the toxin polypeptide precursor is conducted when a lysate of a large volume is processed, and the yield of a target protein can be improved by using methods of multiplex filtration and graded discharge of the waste. The method is especially suitable for a polypeptide with low toxicity. After the completely enclosed separation procedure of the cell lysate, the obtained waste liquid almost contains no toxin polypeptide precursor with low toxicity, with very little toxicity to the operation environment, and can be directly discharged after simple disinfection treatment.

After further combination with a purification procedure, the content of the toxin polypeptide in the finished liquid can be greater than 90%, and the method provided by the present invention is easier to produce and purify the toxin polypeptide as compared with the prior art due to its higher yield, higher purity, and higher safety, and can be used for preparing a toxin polypeptide of high purity on a large scale for clinical applications.

### BRIEF DESCRIPTION OF THE DRAWINGS

FIG. 1 illustrates a schematic process of the method of the present invention.
FIG. 2 illustrates the SDS-PAGE results of the preliminary purification of GSTs-BoNT/A and the further removal of GSTs tags, wherein lane 1 is the GSTs-BoNT/A obtained in the preliminary purification by GSTs affinity chromatographic column, and lane 2 is BoNT/A without GSTs obtained by removing the GSTs tag protein through the digestion of the preliminarily purified protein with Rinovirus 3C Protease.
FIG. 3 illustrates the SDS-PAGE results of a high-purity BoNT/A protein obtained by further purifying the product without GSTs tag through an ion exchange column.
FIG. 4 illustrates the verification results of the dissociation of the double chains of BoNT/A under reducing conditions.

### DETAILED DESCRIPTION

The present invention is further described with reference to the following specific examples, and the advantages and features of the present invention will be clearer as the description proceeds.

### Example 1: Expression of GSTs-BoNT/A

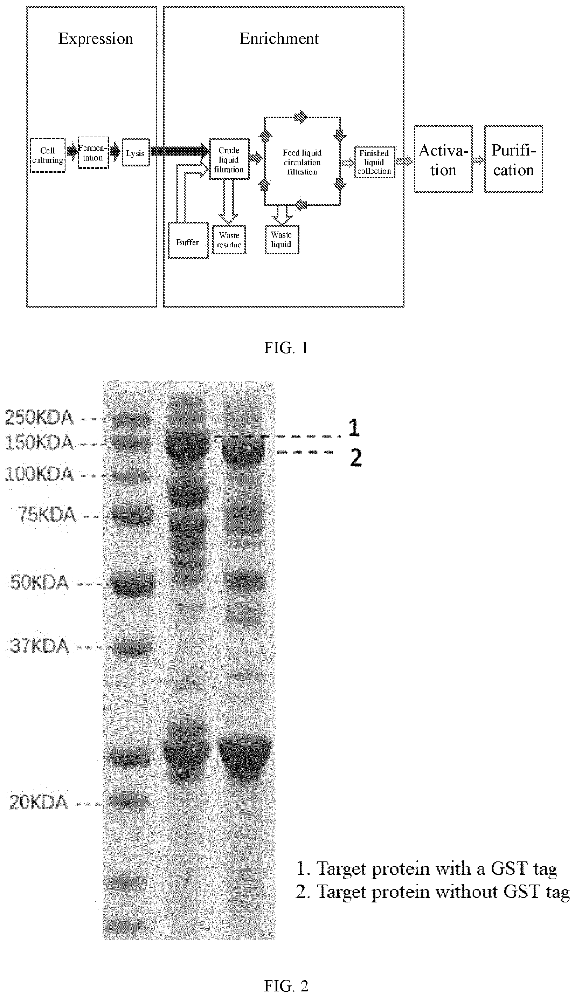

The process of the method of the present invention is shown in FIG. 1, and step 1), expressing a modified toxin polypeptide, is specifically as follows:

### (I): Designing and constructing a nucleic acid molecule encoding the modified toxin polypeptide precursor:

1. the nucleic acid molecule comprises sequentially from the 5' end:
   (a) a nucleotide sequence encoding a glutathione S-transferase is set forth in SEQ ID NO: 1, the glutathione S-transferase encoded by the nucleotide sequence has an amino acid sequence set forth in SEQ ID NO: 2;
   (b) a nucleotide sequence encoding a first protease cleavage site is set forth in SEQ ID NO: 3, the amino acid sequence encoded by the nucleotide sequence is set forth in SEQ ID NO: 4;
   (c) a nucleotide sequence encoding a GS short linker peptide is set forth in SEQ ID NO: 5, which is GGATCC;
   (d) a nucleotide sequence encoding a second polypeptide fragment, comprising nucleotide sequences of the light chain of BoNT/A, a second protease cleavage site, and the heavy chain of BoNT/A, as set forth in SEQ ID NO: 6, the amino acid sequence encoded by the nucleotide sequence is set forth in SEQ ID NO: 7; the nucleotide sequence encoding the second protease cleavage site is set forth in SEQ ID NO: 8, and the amino acid sequence encoded by the nucleotide sequence is set forth in SEQ ID NO: 9.

There can also be no natural loop region between the first functional amino acid structural region and the second functional amino acid structural region. For example, the KTKSLDKGYNK linker sequence between the light chain and the heavy chain may be removed to reduce non-specific protease cleavage.

The nucleotide sequence encoding a toxin polypeptide precursor is set forth in SEQ ID NO: 10, the toxin polypeptide precursor encoded by the nucleotide sequence has an amino acid sequence set forth in SEQ ID NO: 11.

### (II): Constructing a plasmid comprising the nucleic acid molecule of step I

The genetically optimized GSTs-BoNT/A was artificially synthesized in step (I), and NdeI and NotI enzyme cleavage sites were synthesized and added at the two ends thereof. The GSTs-BoNT/A was digested with the NdeI and NotI at 37 °C (New England Biolabs), purified by using a QIquick gel extraction kit (Qiagen), and inserted into NdeI and NotI sites in a pET28a (Novagen) plasmid vector by using a T4 DNA ligase (NEB).

### (III): Transferring the plasmid constructed in step (II) into a host cell

1. Preparation of competent cells: A tube of *E. coli* BL21 DE3 cells (New England Biolabs) was inoculated into a test tube containing 3 mL of LB culture medium and cultured with shaking at 37 °C overnight. On the next day, 0.5 mL of the bacterial culture was inoculated into a 250-mL flask containing 50 mL of LB culture medium and cultured with vigorous shaking (250 rpm) at 37 °C for about 3-5 h. When the OD value of the bacterial colony reached 0.3-0.4 at 600 nm, the flask was transferred to an ice bath and incubated for 10-15 min. The bacterial culture was poured into a 50-mL centrifuge tube and centrifuged at 1000 g at 4 °C for 10 min under an aseptic condition. The supernatant was discarded, and the cells were collected. 10 mL of 0.1 M CaCl₂ was added into the centrifuge tube, and the mixture was mixed well with shaking to resuspend the bacterial cells. The cells were then subjected to an ice bath for 30 min, and centrifuged at 1000 g at 4 °C for 10 min. The supernatant was discarded, and 4 mL of 0.1 M CaCl₂ pre-cooled with ice was added to resuspend the collected bacterial cells. The cells were aliquoted at 0.2 mL per tube and stored at 4 °C for later use within 24 h, and the remaining samples were stored in a low-temperature freezer at -70 °C.
2. Transfection: An appropriate amount of DNA (ng) and the competent *E. coli* were mixed, placed in an ice bath for 15 min, heat-shocked at 42 °C for 30 s, placed in the ice bath for 5 min, shaken at 250 rpm for 1 h in an SOC medium, smeared on a plate with antibiotics, and cultured at 37 °C overnight.

### (IV): Culturing the host cell and inducing the expression of the toxin polypeptide precursor

The composition and ratio of the culture medium: 11.8 g/L of tryptone, 23.6 g/L of yeast extract, 9.4 g/L of K₂HPO₄, 2.2 g/L of KH₂PO₄, and 4 mL/L of glycerol.

The culture condition: The cells were cultured with shaking at 250 rpm at 37 °C overnight.

Inducing expression and fermentation: The cells were transferred to a fermentor and 1 mM IPTG was added to induce the expression at 25 °C for 5 h when the *E. coli* grew to a stage in which the OD₆₀₀ = 1. The OD₆₀₀ threshold may be 0.2-1.5, the temperature threshold may be 37 °C to 10 °C, and the expression time may be 5-16 h.

Cell lysis: The cells were sheared and degassed by conventional methods, in which the cells were lysed to obtain a cell lysate.

### Example 2: Enrichment of GSTs-BoNT/A

Crude liquid filtration: The cell lysate of Example 1 was subjected to a crude liquid filtration as a crude cell lysate with a filter pore size of 0.12-0.65 µm. The liquid that passed through the crude liquid filtration material entered the next filtration procedure as a feed liquid, and the substance that did not pass through the crude liquid filtration material entered the waste residue discharge flow path as a waste residue under the effect of a buffer.

Feed liquid filtration: The liquid after the crude liquid filtration entered a feed liquid filtration step as the feed liquid. The feed liquid was filtered multiple times (at least 2 times) in a circulation pathway, with a filter pore size of below 0.2 µm, to obtain a finished liquid.

After the completely enclosed separation of the cell lysate, the obtained waste liquid almost contained no toxin polypeptide precursor, with very little toxicity to the operation environment, and could be directly discharged after simple disinfection treatment.

### Example 3: Preliminary Purification of GSTs-BoNT/A

The finished liquid obtained in Example 2 further entered a purification module, and the GSTs-BoNT/A was obtained by a conventional affinity chromatography method.

The method is as follows: A chromatographic column was washed with 20 column volumes of a phosphate buffer. GSTs-BoNT/A was eluted with 10 column volumes of a freshly prepared 10 mM glutathione eluent buffer (0.154 g of reduced glutathione dissolved in 50 mL of 50 mM Tris-HCl (pH 8.0)). The elution of the fusion protein was monitored by absorbance reading at 280 nm.

Determination of the proportion of GSTs-BoNT/A in the total protein level: The purified GSTs-BoNT/A was electrophoretically separated at 200 volts by using 4-12% SDS-PAGE (Biorad) and a major band with a molecular weight of 175 kd was GSTs-BoNT/A.

### Example 4: Activation of GSTs-BoNT/A and Dissociation of GSTs

The GSTs-BoNT/A re-adsorbed on a glutathione purification resin chromatographic column was treated using Genscript 3C enzyme. The enzyme cleavage site between GSTs and BoNT/A was cleaved under the effect of the 3C enzyme. GSTs were separated. The enzyme cleavage site between the light chain and the heavy chain of BoNT/A was also cleaved.

The glutathione purification resin chromatographic column was treated with a phosphate buffer. The GSTs tag protein was retained on the column and was thus removed, while the light chain and the heavy chain of BoNT/A were eluted by the phosphate buffer.

Products before and after the removal of the GSTs tag protein were subjected to a conventional SDS-PAGE experiment shown in FIG. 2. Compared with lane 1 in which the tag protein was not cleaved, the GSTs tag protein of lane 1 was cleaved to obtain a BoNT/A molecule without GSTs under the effect of Rinovirus 3C Protease.

### Example 5: Further Purification of BoNT/A

The BoNT/A obtained in Example 4 was further purified by conventional gel filtration chromatography and ion column chromatography to obtain a BoNT/A with a purity of 90% or above.

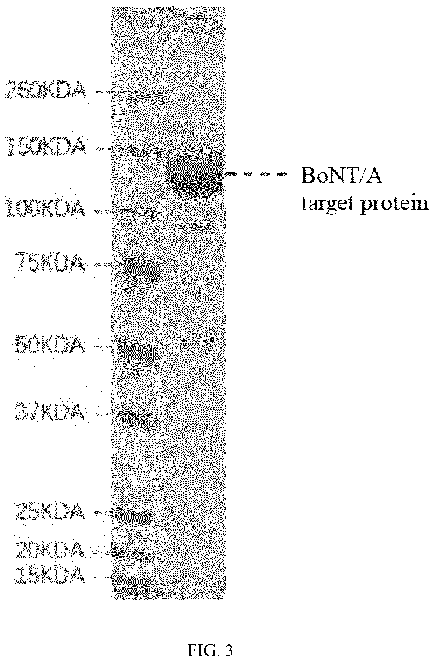

A product after the removal of GSTs was subjected to a conventional SDS-PAGE experiment shown in FIG. 3, and the obtained bands did not include the GSTs tag protein, indicating that the GSTs tag protein had been completely removed. The SDS-PAGE results were scanned, and the purity of the obtained BoNT/A was above 90% by calculation based on the grey density of the band.

GSS, GSGS, and GGSGS polypeptides were adopted to replace a GS part of a short linker peptide, which demonstrated a similar effect to that of GS. The tag protein can be well exposed and thus completely cleaved.

### Example 6: Western Blot Experiment

The products of Example 4 were subjected to a reduction experiment:
A sample was treated by using 100 mM dithiothreitol at 100 °C for 5 min to reduce the sample, and electrophoretically separated at 200 volts by the 4-12% SDS-PAGE (Biorad) to separate the heavy chain and the light chain.

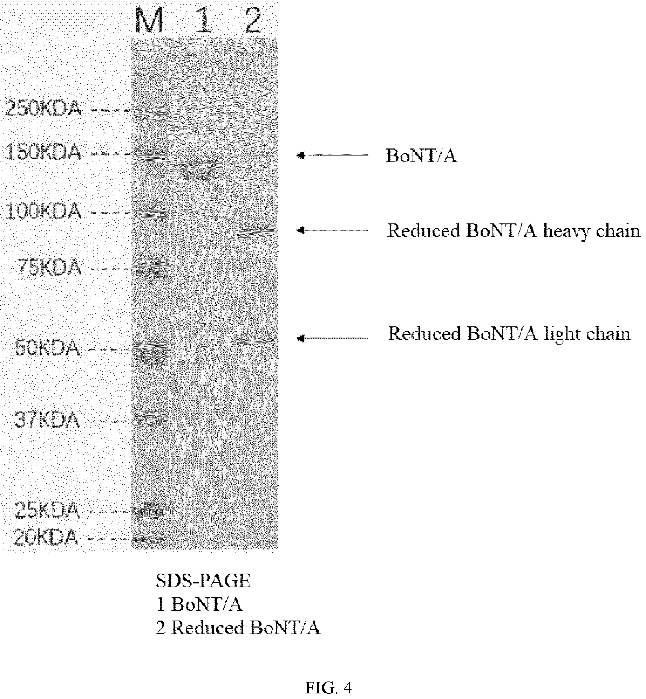

As shown in FIG. 4, the products obtained in Example 4 were subjected to reduction under reducing conditions, and the obtained products were subjected to a conventional SDS-PAGE experiment to obtain two different bands with molecular weights of 100 KDa and 50 KDa, respectively, which demonstrated that the product formed in Example 4 had a dimeric structure in which two peptide fragments were linked by a disulfide bond.

### Example 7: Toxicity Comparison of GSTs-BoNT/A and BoNT/A

The GSTs-BoNT/A obtained in Example 3 had an LD₅₀ of 45-450 ng after intraperitoneal administration in a mice; considering the purity of the injected botulinum toxin protein, the converted LD₅₀ was 22.5-225 ng, with a mid-value of 123.75 ng. The BoNT/A obtained in Example 4 had an LD₅₀ of 0.02-0.05 ng after intraperitoneal administration in mice. Considering the purity of the injected botulinum toxin protein, the converted LD₅₀ was 0.006-0.015 ng, with a mid-value of 0.0105 ng (See Table 1).

**Table 1. Toxicity comparison of GSTs-BoNT/A and BoNT/A molecules in mice biotoxicity study**

| **Test sample** | **Description** | **Dose (ng)** | **Lethality rate of mice 72 h after administration (%)** |
|---|---|---|---|
| GST-BoNT/A. (50% purity) | GST-BoNT/A. (50% purity) | 450 | 100 |
| | | 45 | 50 |
| | | 4.5 | 0 |
| | | 0.45 | 0 |
| BoNT/A (30% purity) | BoNT/A (30% purity) | 0.05 | 100 |
| | | 0.02 | 0 |
| | | 0.01 | 0 |
| | | 0.005 | 0 |

The GSTs-BoNT/A had the activity of botulinum toxin, and the median lethal dose (LD₅₀) thereof was approximately 11786 times higher than the LD₅₀ of the BoNT/A protein after intraperitoneal administration in mice, which indicates that the activity of the toxin precursor molecule of GSTs-BoNT/A recombinant protein is approximately 11786 times weaker than that of the final product BoNT/A. The experiment demonstrates that the toxin precursor molecule of the GSTs-BoNT/A recombinant protein has the activity of botulinum toxin, but toxicity much lower than that of the activated BoNT/A. Due to the ultra-high toxicity of botulinum toxin, high safety operation precautions are required in the manufacturing process even before the activation treatment of a precursor molecule.

The examples of the present application demonstrate that the method claimed in the present application is particularly suitable for the preparation of a genetically recombinant toxin polypeptide, which is activated as a toxin molecule with toxicity only by hydrolysis with a specific protease, and is expressed in the form of a mildly toxic precursor in the host cell and present in the form of the mildly toxic precursor in the cell lysate. In this case, the advantage that the enrichment of the polypeptide precursor is conducted in an enclosed system in the method avoids the tedious and costly implementation of isolation facilities, for example, an isolator, when the toxin polypeptide is industrially produced, such that the preparation method is more suitable for the large-scale industrial production of the polypeptide.

## Claims

1. A method for preparing a toxin polypeptide, comprising: step 1), expressing a toxin polypeptide precursor; step 2), enriching the toxin polypeptide precursor; and step 3), activating the toxin polypeptide precursor to obtain the toxin polypeptide;
wherein the toxin polypeptide precursor comprises:
(I) the first polypeptide fragment, comprising:
(a) the tag protein;
(b) the structural region comprising the first protease cleavage site;
(c) the short linker peptide;
(II) the second polypeptide fragment, comprising:
(d) the first functional amino acid structural region, comprising the metal ion-dependent protease activity domain;
(e) the structural region comprising the second protease cleavage site;
(f) the second functional amino acid structural region, comprising the receptor-binding domain that can bind to the surface receptor of the target cell and/or the translocation domain that can mediate the transfer of the polypeptide across the vesicle membrane;
wherein the protease that specifically recognizes the first protease cleavage site and the protease that specifically recognizes the second protease cleavage site are both the proteases of rhinoviruses;
wherein the first protease cleavage site and the second protease cleavage site are both LEVLFQGP;
wherein the second enzyme cleavage site is embedded into, or partially replaces or completely replaces a natural loop region between a first functional peptide fragment and a second functional peptide fragment.

2. The method according to claim 1, wherein the embedding refers to an embedding between two certain amino acids in the loop region; the partial replacement refers to that the second enzyme cleavage site replaces part of the amino acid sequence of the loop region; the complete replacement refers to that the amino acid sequence of the natural loop region is completely replaced by the second enzyme cleavage site.

3. The method according to claim 1, wherein the first functional amino acid structural region of the toxin polypeptide precursor comprises a Zn²⁺ protease binding domain of the light chain of clostridial neurotoxin, the receptor-binding domain of the second functional amino acid structural region is a cell surface antigen-binding domain of the heavy chain of clostridial neurotoxin, and the translocation domain of the second functional amino acid structural region that mediates the transfer of the polypeptide across the vesicle membrane is a domain that mediates the transfer of clostridial neurotoxin across the vesicle membrane;
Preferably, wherein the clostridial neurotoxin is a botulinum neurotoxin or tetanus toxin.

4. The method according to claim 1, wherein the second polypeptide fragment has an amino acid sequence comprising a fragment set forth in SEQ ID NO: 7.

5. The method according to claim 1, wherein the tag protein is selected from the following proteins: a glutathione S-transferase (GSTs), C-myc, chitin-binding domain, maltose-binding protein (MBP), SUMO heterologous affinity moiety, monoclonal antibody or protein A, streptavidin-binding protein (SBP), cellulose-binding domain, calmodulin-binding peptide, S tag, Strep tag II, FLA, protein A, protein G, histidine affinity tag (HAT), or polyhistidine.

6. The method according to claim 1, wherein the toxin polypeptide precursor sequentially comprises, from the N terminus: the glutathione S-transferase, LEVLFQGPLGS, the light chain of BoNT/A, LEVLFQGP, and the heavy chain of BoNT/A;
preferably, wherein the toxin polypeptide precursor has an amino acid sequence set forth in SEQ ID NO: 11.

7. The method according to any one of claims 1-6, wherein the step 1) comprises: (1) designing a nucleic acid molecule encoding the toxin polypeptide precursor; (2) constructing a vector comprising the nucleic acid molecule; (3) transferring the nucleic acid vector into a suitable host cell; and (4) culturing the host cell, and allowing or inducing the host cell to express the toxin polypeptide precursor encoded by the nucleic acid vector.

8. The method according to claim 7, wherein the nucleic acid molecule comprises sequentially from the 5' end:
(a) a nucleotide sequence encoding a glutathione S-transferase is set forth in SEQ ID NO: 1, the glutathione S-transferase encoded by the nucleotide sequence has an amino acid sequence set forth in SEQ ID NO: 2;
(b) a nucleotide sequence encoding a first protease cleavage site is set forth in SEQ ID NO: 3, the amino acid sequence encoded by the nucleotide sequence is set forth in SEQ ID NO: 4;
(c) a nucleotide sequence encoding a GS short linker peptide is set forth in SEQ ID NO: 5, which is GGATCC;
(d) a nucleotide sequence encoding a second polypeptide fragment, comprising nucleotide sequences of the light chain of BoNT/A, a second protease cleavage site, and the heavy chain of BoNT/A, as set forth in SEQ ID NO: 6, the amino acid sequence encoded by the nucleotide sequence is set forth in SEQ ID NO: 7; the nucleotide sequence encoding the second protease cleavage site is set forth in SEQ ID NO: 8, and the amino acid sequence encoded by the nucleotide sequence is set forth in SEQ ID NO: 9.

9. The method according to claim 7, wherein the nucleotide sequence encoding a toxin polypeptide precursor is set forth in SEQ ID NO: 10.

10. The method according to claim 7, wherein the step 1) further comprises: (5) fermenting a cell capable of expressing the toxin polypeptide precursor; preferably, the step 1) further comprises: (6) lysing the cell expressing the toxin polypeptide to obtain a cell lysate comprising the toxin polypeptide precursor.

11. The method according to any one of claims 1-10, wherein the step 2) comprises enriching the toxin polypeptide precursor by conducting a multiplex filtration procedure.

12. The method according to claim 11, wherein the multiplex filtration comprises a crude liquid filtration and a feed liquid circulation filtration;
Preferably, wherein a material of the crude liquid filtration has a pore size of 0. 1-0.65 µm;
Preferably, wherein the feed liquid circulation filtration step comprises filtering a feed multiple times, and a material of the feed liquid circulation filtration has a pore size of 0.2 µm or less.

13. The method according to any one of claims 1-12, wherein the step 3) comprises digesting the toxin polypeptide precursor by using a protease to obtain the toxin polypeptide;
Preferably, wherein the toxin polypeptide precursor forms at least one dimer structure after the protease digestion.

14. The method according to any one of claims 1-13, wherein the toxin polypeptide precursor has low toxicity relative to the toxin polypeptide.

15. The method according to any one of claims 1-14, wherein the method further comprises step 4), purifying the toxin polypeptide.

## Patentansprüche

1. Verfahren zur Herstellung eines Toxinpolypeptids, umfassend: Schritt 1), Exprimieren eines Toxinpolypeptid-Vorläufers; Schritt 2), Anreichern des Toxinpolypeptid-Vorläufers; und Schritt 3), Aktivieren des Toxinpolypeptid-Vorläufers, um das Toxinpolypeptid zu erhalten;
wobei der Toxinpolypeptid-Vorläufer Folgendes umfasst:
(I) das erste Polypeptidfragment, das Folgendes umfasst:
(a) das Tag-Protein;
(b) die Strukturregion, die die erste Proteasespaltstelle umfasst;
(c) das Short-Linker-Peptid;
(II) das zweite Polypeptidfragment, das Folgendes umfasst:
(d) die erste funktionelle Aminosäurestrukturregion, die die Metallionen-abhängige Proteaseaktivitätsdomäne umfasst;
(e) die Strukturregion, die die zweite Proteasespaltstelle umfasst;
(f) die zweite funktionelle Aminosäurestrukturregion, die die Rezeptorbindungsdomäne, die an den Oberflächenrezeptor der Zielzelle binden kann, und/oder die Translokationsdomäne, die den Transfer des Polypeptids über die Vesikelmembran vermitteln kann, umfasst;
wobei die Protease, die die erste Proteasespaltstelle spezifisch erkennt, und die Protease, die die zweite Proteasespaltstelle spezifisch erkennt, beide die Proteasen von Rhinoviren sind;
wobei die erste Proteasespaltstelle und die zweite Proteasespaltstelle beide LEVLFQGP sind;
wobei die zweite Enzymspaltstelle in eine natürliche Schleifenregion zwischen einem ersten funktionellen Peptidfragment und einem zweiten funktionellen Peptidfragment eingebettet ist oder diese teilweise ersetzt oder vollständig ersetzt.

2. Verfahren nach Anspruch 1, wobei sich das Einbetten auf ein Einbetten zwischen zwei bestimmten Aminosäuren in der Schleifenregion bezieht; der partielle Ersatz sich darauf bezieht, dass die zweite Enzymspaltstelle einen Teil der Aminosäuresequenz der Schleifenregion ersetzt; der vollständige Ersatz sich darauf bezieht, dass die Aminosäuresequenz der natürlichen Schleifenregion vollständig durch die zweite Enzymspaltstelle ersetzt wird.

3. Verfahren nach Anspruch 1, wobei die erste funktionelle Aminosäurestrukturregion des Toxinpolypeptid-Vorläufers eine Zn²⁺-Protease-Bindungsdomäne der leichten Kette von Clostridien-Neurotoxin umfasst, die Rezeptorbindungsdomäne der zweiten funktionellen Aminosäurestrukturregion eine Zelloberflächenantigenbindungsdomäne der schweren Kette von Clostridien-Neurotoxin ist und die Translokationsdomäne der zweiten funktionellen Aminosäurestrukturregion, die den Transfer des Polypeptids über die Vesikelmembran vermittelt, eine Domäne ist, die den Transfer von Clostridien-Neurotoxin über die Vesikelmembran vermittelt;
Vorzugsweise wobei das Clostridien-Neurotoxin ein Botulinum-Neurotoxin oder Tetanus-Toxin ist.

4. Verfahren nach Anspruch 1, wobei das zweite Polypeptidfragment eine Aminosäuresequenz aufweist, die ein Fragment umfasst, das in SEQ **ID** NO: 7 dargelegt ist.

5. Verfahren nach Anspruch 1, wobei das Tag-Protein ausgewählt ist aus den folgenden Proteinen: eine Glutathion-S-Transferase (GSTs), C-myc, Chitin-bindende Domäne, Maltose-bindendes Protein (MBP), SUMO-heterologe Affinitätseinheit, monoklonaler Antikörper oder Protein A, Streptavidin-bindendes Protein (SBP), Cellulose-bindende Domäne, Calmodulinbindendes Peptid, S-Tag, Strep-Tag II, FLA, Protein A, Protein G, Histidin-Affinitäts-Tag (HAT) oder Polyhistidin.

6. Verfahren nach Anspruch 1, wobei der Toxinpolypeptid-Vorläufer vom N-Terminus aus sequentiell Folgendes umfasst: die Glutathion-S-Transferase, LEVLFQGPLGS, die leichte Kette von BoNT/A, LEVLFQGP und die schwere Kette von BoNT/A;
vorzugsweise wobei der Toxinpolypeptid-Vorläufer eine Aminosäuresequenz aufweist, die in SEQ ID NO: 11 dargelegt ist.

7. Verfahren nach einem der Ansprüche 1-6, wobei der Schritt 1) Folgendes umfasst: (1) Entwerfen eines Nukleinsäuremoleküls, das den Toxinpolypeptid-Vorläufer kodiert; (2) Konstruieren eines Vektors, der das Nukleinsäuremolekül umfasst; (3) Übertragen des Nukleinsäure-Vektors in eine geeignete Wirtszelle; und (4) Kultivieren der Wirtszelle und Ermöglichen oder Induzieren, dass die Wirtszelle den Toxinpolypeptid-Vorläufer, der durch die Nukleinsäure-Vektor kodiert wird, exprimiert.

8. Verfahren nach Anspruch 7, wobei das Nukleinsäuremolekül sequentiell vom 5'-Ende aus Folgendes umfasst:
(a) eine Nukleotidsequenz, die eine Glutathion-S-Transferase kodiert, ist in SEQ ID NO: 1 dargelegt, die Glutathion-S-Transferase, die durch die Nukleotidsequenz kodiert wird, weist eine Aminosäuresequenz auf, die in SEQ ID NO: 2 dargelegt ist;
(b) eine Nukleotidsequenz, die eine erste Proteasespaltstelle kodiert, ist in SEQ ID NO: 3 dargelegt, die Aminosäuresequenz, die durch die Nukleotidsequenz kodiert wird, ist in SEQ ID NO: 4 dargelegt;
(c) eine Nukleotidsequenz, die ein GS-Short-Linker-Peptid kodiert, ist in SEQ ID NO: 5 dargelegt, bei der es sich um GGATCC handelt;
(d) eine Nukleotidsequenz, die ein zweites Polypeptidfragment kodiert, umfassend Nukleotidsequenzen der leichten Kette von BoNT/A, eine zweite Proteasespaltstelle und die schwere Kette von BoNT/A, wie in SEQ ID NO: 6 dargelegt, die Aminosäuresequenz, die durch die Nukleotidsequenz kodiert wird, ist in SEQ ID NO: 7 dargelegt; die Nukleotidsequenz, die die zweite Proteasespaltstelle kodiert, ist in SEQ ID NO: 8 dargelegt und die Aminosäuresequenz, die durch die Nukleotidsequenz kodiert wird, ist in SEQ ID NO: 9 dargelegt.

9. Verfahren nach Anspruch 7, wobei die Nukleotidsequenz, die einen Toxinpolypeptid-Vorläufer kodiert, in SEQ ID NO: 10 dargelegt ist.

10. Verfahren nach Anspruch 7, wobei der Schritt 1) ferner Folgendes umfasst: (5) Fermentieren einer Zelle, die in der Lage ist, den Toxinpolypeptid-Vorläufer zu exprimieren; wobei vorzugsweise der Schritt 1) ferner Folgendes umfasst: (6) Lysieren der Zelle, die das Toxinpolypeptid exprimiert, um ein Zelllysat zu erhalten, das den Toxinpolypeptid-Vorläufer umfasst.

11. Verfahren nach einem der Ansprüche 1-10, wobei der Schritt 2) Anreichern des Toxinpolypeptid-Vorläufers durch Durchführen eines Multiplexfiltrationsvorgangs umfasst.

12. Verfahren nach Anspruch 11, wobei die Multiplexfiltration eine Rohflüssigkeitsfiltration und eine Zulaufflüssigkeitszirkulationsfiltration umfasst;
Vorzugsweise wobei ein Material der Rohflüssigkeitsfiltration eine Porengröße von 0,1-0,65 µm aufweist;
Vorzugsweise wobei der Zulaufflüssigkeitszirkulationsfiltrationsschritt mehrfaches Filtern eines Zulaufs umfasst und ein Material der Zulaufflüssigkeitszirkulationsfiltration eine Porengröße von 0,2 µm oder weniger aufweist.

13. Verfahren nach einem der Ansprüche 1-12, wobei der Schritt 3) Abbauen des Toxinpolypeptid-Vorläufers unter Verwendung einer Protease zum Erhalten des Toxinpolypeptids umfasst;
Vorzugsweise wobei der Toxinpolypeptid-Vorläufer nach dem Proteaseabbauen mindestens eine Dimerstruktur ausbildet.

14. Verfahren nach einem der Ansprüche 1-13, wobei der Toxinpolypeptid-Vorläufer eine geringe Toxizität relativ zu dem Toxinpolypeptid aufweist.

15. Verfahren nach einem der Ansprüche 1-14, wobei das Verfahren ferner Schritt 4), Reinigen des Toxinpolypeptids, umfasst.

## Revendications

1. Procédé permettant la préparation d'un polypeptide de toxine, comprenant : étape 1), l'expression d'un précurseur de polypeptide de toxine ; étape 2), l'enrichissement du précurseur de polypeptide de toxine ; et étape 3), l'activation du précurseur de polypeptide de toxine pour obtenir le polypeptide de toxine ;
dans lequel le précurseur de polypeptide de toxine comprend :
(I) le premier fragment polypeptidique, comprenant :
(a) l'étiquette protéique ;
(b) la région structurale comprenant le premier site de clivage protéolytique ;
(c) le peptide de liaison court ;
(II) le second fragment polypeptidique, comprenant :
(d) la première région structurale d'acides aminés fonctionnelle, comprenant le domaine d'activité de protéase dépendant des ions métalliques ;
(e) la région structurale comprenant le second site de clivage protéolytique ;
(f) la seconde région structurale d'acides aminés fonctionnelle, comprenant le domaine de liaison au récepteur capable de se lier au récepteur de surface de la cellule cible et/ou le domaine de translocation capable de médier le transfert du polypeptide à travers la membrane vésiculaire ;
dans lequel la protéase qui reconnaît spécifiquement le premier site de clivage protéolytique et la protéase qui reconnaît spécifiquement le second site de clivage protéolytique sont toutes deux des protéases de rhinovirus ;
dans lequel le premier site de clivage protéolytique et le second site de clivage protéolytique sont tous deux LEVLFQGP ;
dans lequel le second site de clivage enzymatique est incorporé dans, ou remplace partiellement ou remplace complètement une région de boucle naturelle entre un premier fragment peptidique fonctionnel et un second fragment peptidique fonctionnel.

2. Procédé selon la revendication 1, dans lequel l'incorporation fait référence à une incorporation entre deux certains acides aminés dans la région de boucle ; le remplacement partiel fait référence au fait que le second site de clivage enzymatique remplace une partie de la séquence d'acides aminés de la région de boucle ; le remplacement complet fait référence au fait que la séquence d'acides aminés de la région de boucle naturelle est complètement remplacée par le second site de clivage enzymatique.

3. Procédé selon la revendication 1, dans lequel la première région structurale d'acides aminés fonctionnelle du précurseur de polypeptide de toxine comprend un domaine de liaison à la protéase Zn²⁺ de la chaîne légère de la neurotoxine clostridiale, le domaine de liaison au récepteur de la seconde région structurale d'acides aminés fonctionnelle est un domaine de liaison à un antigène de surface cellulaire de la chaîne lourde de la neurotoxine clostridiale, et le domaine de translocation de la seconde région structurale d'acides aminés fonctionnelle qui assure la médiation du transfert du polypeptide à travers la membrane vésiculaire est un domaine qui assure la médiation du transfert de la neurotoxine clostridiale à travers la membrane vésiculaire ;
de préférence, dans lequel la neurotoxine clostridiale est une neurotoxine botulinique ou une toxine tétanique.

4. Procédé selon la revendication 1, dans lequel le second fragment polypeptidique présente une séquence d'acides aminés comprenant un fragment présenté dans SEQ ID N° : 7.

5. Procédé selon la revendication 1, dans lequel l'étiquette protéique est choisie parmi les protéines suivantes : une glutathion S-transférase (GSTs), C-myc, un domaine de liaison à la chitine, une protéine de liaison au maltose (MBP), une fraction d'affinité hétérologue SUMO, un anticorps monoclonal ou une protéine A, une protéine de liaison à la streptavidine (SBP), un domaine de liaison à la cellulose, un peptide de liaison à la calmoduline, une étiquette S, une étiquette Strep II, FLA, une protéine A, une protéine G, une étiquette d'affinité à l'histidine (HAT) ou une polyhistidine.

6. Procédé selon la revendication 1, dans lequel le précurseur de polypeptide de toxine comprend séquentiellement, à partir de l'extrémité N : la glutathion S-transférase, LEVLFQGPLGS, la chaîne légère de BoNT/A, LEVLFQGP, et la chaîne lourde de BoNT/A ;
de préférence, dans lequel le précurseur de polypeptide de toxine présente une séquence d'acides aminés présentée dans SEQ ID N° : 11.

7. Procédé selon l'une quelconque des revendications 1 à 6, dans lequel l'étape 1) comprend : (1) la conception d'une molécule d'acide nucléique codant pour le précurseur de polypeptide de toxine ; (2) la construction d'un vecteur comprenant la molécule d'acide nucléique ; (3) le transfert du vecteur d'acide nucléique dans une cellule hôte appropriée ; et (4) la culture de la cellule hôte, et le fait de permettre ou d'induire la cellule hôte à exprimer le précurseur de polypeptide de toxine codé par le vecteur d'acide nucléique.

8. Procédé selon la revendication 7, dans lequel la molécule d'acide nucléique comprend séquentiellement à partir de l'extrémité 5' :
(a) une séquence nucléotidique codant pour une glutathion S-transférase présentée dans SEQ ID N° : 1, la glutathion S-transférase codée par la séquence nucléotidique présente une séquence d'acides aminés présentée dans SEQ ID N° : 2 ;
(b) une séquence nucléotidique codant pour un premier site de clivage protéolytique est présentée dans SEQ ID N° : 3, la séquence d'acides aminés codée par la séquence nucléotidique est présentée dans SEQ ID N° : 4 ;
(c) une séquence nucléotidique codant pour un peptide de liaison court GS est présentée dans SEQ ID N° : 5, qui est GGATCC ;
(d) une séquence nucléotidique codant pour un second fragment polypeptidique, comprenant des séquences nucléotidiques de la chaîne légère de BoNT/A, un second site de clivage protéolytique et la chaîne lourde de BoNT/A, telle que présentée dans SEQ ID N° : 6, la séquence d'acides aminés codée par la séquence nucléotidique est présentée dans SEQ ID N° : 7 ; la séquence nucléotidique codant pour le second site de clivage protéolytique est présentée dans SEQ ID N° : 8, et la séquence d'acides aminés codée par la séquence nucléotidique est présentée dans SEQ ID N° : 9.

9. Procédé selon la revendication 7, dans lequel la séquence nucléotidique codant pour un précurseur de polypeptide de toxine est présentée dans SEQ ID N° : 10.

10. Procédé selon la revendication 7, dans lequel l'étape 1) comprend en outre : (5) la fermentation d'une cellule capable d'exprimer le précurseur de polypeptide de toxine ; de préférence, l'étape 1) comprend en outre : (6) la lyse de la cellule exprimant le polypeptide de toxine pour obtenir un lysat cellulaire comprenant le précurseur de polypeptide de toxine.

11. Procédé selon l'une quelconque des revendications 1 à 10, dans lequel l'étape 2) comprend l'enrichissement du précurseur de polypeptide de toxine en effectuant une procédure de filtration multiplex.

12. Procédé selon la revendication 11, dans lequel la filtration multiplex comprend une filtration de liquide brut et une filtration par circulation de liquide d'alimentation ;
de préférence, dans lequel un matériau de la filtration de liquide brut présente une taille de pores de 0,1 à 0,65 µm ;
de préférence, dans lequel l'étape de filtration par circulation de liquide d'alimentation comprend la filtration d'une alimentation plusieurs fois, et un matériau de la filtration par circulation de liquide d'alimentation présente une taille de pores de 0,2 µm ou moins.

13. Procédé selon l'une quelconque des revendications 1 à 12, dans lequel l'étape 3) comprend la digestion du précurseur de polypeptide de toxine en utilisant une protéase pour obtenir le polypeptide de toxine ;
de préférence, dans lequel le précurseur de polypeptide de toxine forme au moins une structure dimère après la digestion par la protéase.

14. Procédé selon l'une quelconque des revendications 1 à 13, dans lequel le précurseur de polypeptide de toxine présente une faible toxicité par rapport au polypeptide de toxine.

15. Procédé selon l'une quelconque des revendications 1 à 14, dans lequel le procédé comprend en outre l'étape 4) de purification du polypeptide de toxine.
